# EUROPEAN PATENT APPLICATION

(11) **EP 2 361 904 A1**
(43) Date of publication of application: **31.08.2011**
(21) Application number: 10382025.4
(22) Date of filing: 04.02.2010
(51) Int. Cl.: C07D 231/22, A61K 31/4155, A61P 25/00

(54) **4-[-2-[[5-methyl-1-(2-naphtalenyl)-1h-pyrazol-3-yl]oxy]ethyl]morpholine hydrochloride and crystalline forms thereof**

(71) Applicant: Laboratorios del. Dr. Esteve, S.A., 08041 Barcelona (ES)
(72) Inventor: Berenguer Maimó, Ramón, 08001 Barcelona (ES); Medrano Rupérez, Jorge, 08921 Santa Coloma de Bramenet - Barcelona (ES); Cuberes-Altisent, Ma Rosa, 08172-San Cugat del Vallés Barcelona (ES)
(74) Representative: ABG Patentes, S.L.

(57) **Abstract**

The present invention relates to 4-[2-[[5-methyl-1-(2-naphthalenyl)-1 H-pyrazol-3-yl]oxy]ethyl]morpholine hydrochloride, a crystalline form thereof and processes for their preparation.

## Description

### FIELD OF THE INVENTION

The present invention relates to the hydrochloride salt of 4-[2-[[5-methyl-1-(2-naphthalenyl)-1 H-pyrazol-3-yl]oxy]ethyl]morpholine (P027), its crystalline form and processes for their preparation.

### BACKGROUND

The search for new therapeutic agents has been greatly aided in recent years by better understanding of the structure of proteins and other biomolecules associated with target diseases. One important class of these proteins is the sigma (σ) receptor, a cell surface receptor of the central nervous system (CNS) which may be related to the dysphoric, hallucinogenic and cardiac stimulant effects of opioids. From studies of the biology and function of sigma receptors, evidence has been presented that sigma receptor ligands may be useful in the treatment of psychosis and movement disorders such as dystonia and tardive dyskinesia, and motor disturbances associated with Huntington's chorea or Tourette's syndrome and in Parkinson's disease (Walker, J.M. et al, Pharmacological Reviews, 1990, 42, 355). It has been reported that the known sigma receptor ligand rimcazole clinically shows effects in the treatment of psychosis (Snyder, S.H., Largent, B.L. J. Neuropsychiatry 1989, 1, 7). The sigma binding sites have preferential affinity for the dextrorotatory isomers of certain opiate benzomorphans, such as (+)SKF 10047, (+)cyclazocine, and (+)pentazocine and also for some narcoleptics such as haloperidol.

The sigma receptor has at least two subtypes, which may be discriminated by stereoselective isomers of these pharmacoactive drugs. SKF 10047 has nanomolar affinity for the sigma 1 (σ-1) site, and has micromolar affinity for the sigma 2 (σ-2) site. Haloperidol has similar affinities for both subtypes. Endogenous sigma ligands are not known, although progesterone has been suggested to be one of them. Possible sigma-site-mediated drug effects include modulation of glutamate receptor function, neurotransmitter response, neuroprotection, behavior, and cognition (Quirion, R. et al. Trends Pharmacol. Sci., 1992, 13:85-86). Most studies have implied that sigma binding serve as link between the three systems.

In view of the potential therapeutic applications of agonists or antagonists of the sigma receptor, a great effort has been directed to find selective ligands. Thus, the prior art discloses different sigma receptor ligands. 4-[2-[[5-methyl-1-(2-naphthalenyl)-1H-pyrazol-3-yl]oxy]ethyl]morpholine is one of such promising sigma receptor ligands. The compound and its synthesis are disclosed and claimed in WO2006/021462.

4-[2-[[5-methyl-1-(2-naphthalenyl)-1H-pyrazol-3-yl]oxy]ethyl]morpholine is a highly selective sigma-1 (σ-1) receptor antagonist. It has displayed strong analgesic activity in the treatment and prevention of chronic and acute pain, and particularly, neuropathic pain. The compound has a molecular weight 337.42 uma. The structural formula of the compound is:

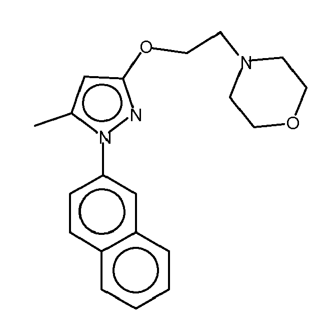

To carry out its pharmaceutical development and reealise its potential, there is a need in the art for additional forms of 4-[2-[[5-methyl-1-(2-naphthalenyl)-1 H-pyrazol-3-yl]oxy]ethyl]morpholine that will facilitate the preparation of better formulations of this active pharmaceutical ingredient. Furthermore, new forms of the compound may also improve its production, handling and storage characteristics and its therapeutic effects such as pharmacological properties.

In this regard, alternative morphological forms of the compound may have widely different properties such as, for example, enhanced thermodynamic stability, higher purity or improved bioavailability (e.g. better absorption, dissolution patterns). Specific compound forms could also facilitate the manufacturing (e.g. enhanced flowability), handling and storage (e.g. non-hygroscopic, long shelf life) of the compound formulations or allow the use of a lower dose of the therapeutic agent, thus decreasing its potential side effects. Thus it is important to provide such forms, having desirable properties for pharmaceutical use.

### BRIEF DESCRIPTION OF THE INVENTION

The inventors of the present invention have surprisingly found and demonstrated that the hydrochloride salt of 4-[2-[[5-methyl-1-(2-naphthalenyl)-1 H-pyrazol-3-yl]oxy]ethyl]morpholine (P027), as well as a novel and highly stable crystalline form of the P027 compound (phase I) provides advantageous production, handling, storage and therapeutic properties. The novel P027 phase I is stable over the time and has good flow and dissolution characteristics.

The P027 compound has a molecular weight 373.88 uma, a pKa of 6.73 and a melting point of 194.2 °C. The compound is very soluble in water and freely soluble in methanol, 1 N hydrochloric acid and dimethyl sulphoxide. It is sparingly soluble in ethanol, slightly soluble in acetone and practically insoluble in ethyl acetate and in 1 N sodium hydroxide. The product exhibits a better dissolution and absorption profile in vivo than its related base.

In one embodiment, the present invention is directed to a hydrochloride salt 4-[2-[[5-methyl-1-(2-naphthalenyl)-1 H-pyrazol-3-yl]oxy]ethyl]morpholine.

In another embodiment, the present invention refers to a crystalline form of 4-[2-[[5-methyl-1-(2-naphthalenyl)-1 H-pyrazol-3-yl]oxy]ethyl]morpholine hydrochloride.

Preferably, said crystalline form is characterized by a X-ray powder diffraction pattern having characteristic peaks at a reflection angle [2θ] of about 5.9, 8.1, 11.3, 11.7, 14.2, 15.1, 15.8, 16.3, 16.8, 17.8, 18.1, 18.6, 19.8, 20.9, 21.9, 22.8, 23.0, 23.2, 23.6, 23.9, 24.3, 25.0, 25.1, 28.0, 28.3, 28.6, 29.0, 29.2, 30.7, and 30.9, with the 2θ values being obtained using copper radiation (Cu_{kα1}1.54060Å).

According to another embodiment, the crystalline form of 4-[2-[[5-methyl-1-(2-naphthalenyl)-1H-pyrazol-3-yl]oxy]ethyl]morpholine hydrochloride according to the present invention has a monoclinic unit cell with the following approximate dimensions:
a = 29.4(3) A
b=11.7(11)Å
c=11.0(10)Å
α = 90°
β=91.3(2)
γ=90°

The hydrochloride salt of 4-[2-[[5-methyl-1-(2-naphthalenyl)-1 H-pyrazol-3-yl]oxy]ethyl]morpholine (P027) can be prepared by adding an hydrochloric acid solution to its corresponding base dissolved in the appropriate solvent.

The P027 phase I form can be prepared by crystallizing the P027 compound in various solvents by means of various techniques such as: solvent evaporation at varying temperatures, crystallization from hot saturated solutions, crystallization by antisolvent addition, crystallization by antisolvent diffusion, crystallization from water and solvents mixtures and the preparation of suspensions.

In one embodiment of the present invention, the P027 phase I form is obtained by dissolving the P027 compound in a suitable solvent and then evaporating the solvent to obtain the phase I crystalline form. According to one variant of this process, the P027 compound is dissolved at a temperature ranging from about room temperature to about 120 °C. In another variant to this process, the solvent is evaporated at a temperature ranging from about -21 °C to about 60 °C. In a further variant of this process, the P027 solution is allowed to cool down slowly. In yet another variant of this process, the P027 solution is cooled down rapidly.

In another embodiment of the present invention, the P027 phase I form is obtained by mixing a P027 solution and an antisolvent. In a variant of this process, the P027 solution is added to the antisolvent. In another variant of this process, the antisolvent is added to the P027 solution. In an additional variant of this process the P027 solution and the antisolvent are mixed at a temperature ranging from about room temperature to about 90 °C.

In an additional embodiment of the present invention, the P027 phase I form is obtained by combining a P027 solution and an antisolvent through diffusion. In a variant of this process, the diffusion is a liquid-liquid diffusion. In another variant of this process, the diffusion is a gas-liquid diffusion.

In another embodiment of the present invention, the P027 phase I form is collected from mixtures of P027, water and solvents.

In yet an additional embodiment of the present invention, the P027 phase I form is obtained from suspensions containing the P027 compound. In variant of this process, the suspension is maintained at a temperature ranging from about room temperature to about 80 °C.

In an additional embodiment of the present invention, an hydrochloric acid solution and 4-[2-[[5-methyl-1-(2-naphthalenyl)-1 H-pyrazol-3-yl]oxy]ethyl]morpholine are mixed to obtain the P027 compound. Preferably, an antisolvent is added to the mixture to induce the formation of the P027 compound.

Various of the embodiments above may require additional steps, such as centrifugation, to further isolate the P027 phase I form.

A further embodiment of the present invention includes pharmaceutical compositions containing 4-[2-[[5-methyl-1-(2-naphthalenyl)-1 H-pyrazol-3-yl]oxy]ethyl]morpholine hydrochloride, a crystalline form of the P027 compound or the P027 phase I.

These aspects and preferred embodiments thereof are additionally also defined in the claims.

### BRIEF DESCRIPTION OF THE FIGURES

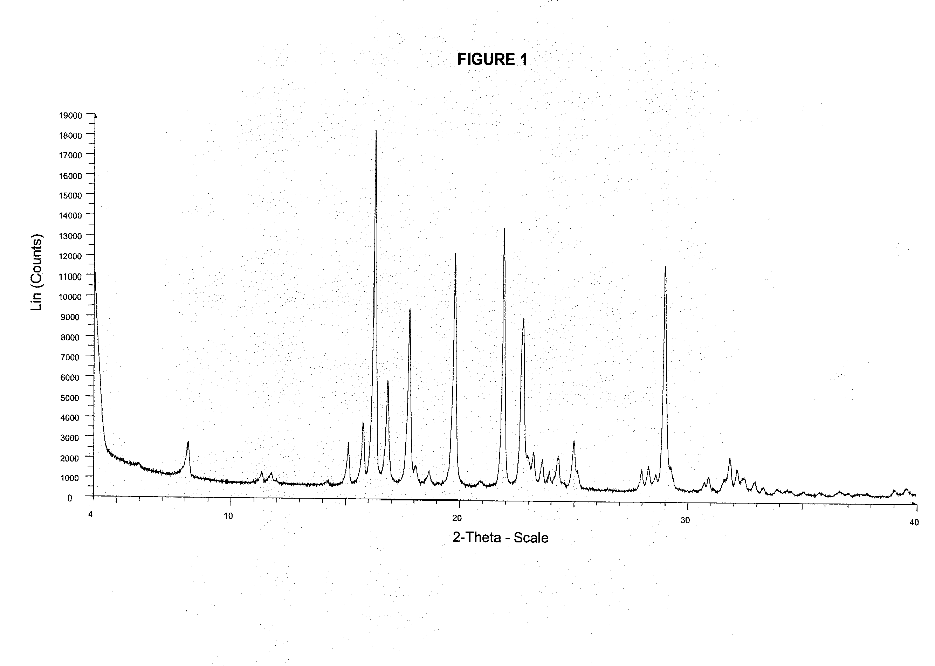

**Fig. 1**: Standard PXRD pattern of phase I.

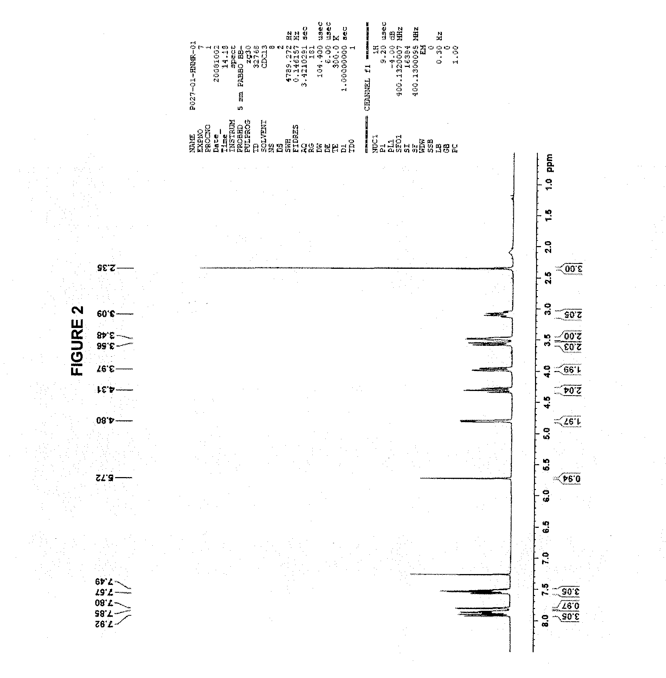

**Fig. 2**:¹H NMR spectrum of a P027 compound solution.
**Fig. 3**: DSC and TGA analyses of phase I.
**Fig. 4**: FTIR analysis of phase I.
**Fig. 5**: Randomly selected PXRD patterns of different solids corresponding to phase I in which texture effects can be observed.
**Fig. 6**: PXRD pattern of phase I samples before and after grinding. The standard PXRD pattern of phase I is shown for comparison purposes.
**Fig. 7**:¹H-NMR spectra of the samples depicted in Figure 6.
**Fig. 8**: DSC analysis of phase I at a heating rate of 5 °C/min.
**Fig. 9**: DSC analysis of phase I at a heating rate of 20 °C/min.
**Fig. 10**: Ortep-Plot (50 %) showing the organic cation and the two independent half chlorine anions contained in the unit cell.
**Fig. 11**: Ortep-Plot (50 %) showing the structure of phase I. The hydrogen bonds are marked with discontinuous lines.
**Fig. 12**: Simulated powder diffraction pattern generated from the single crystal data of phase I.
**Fig. 13**: Comparison of the simulated powder diffraction pattern obtained from single crystal data and the experimentally measured powder diffraction pattern of phase I.
**Fig. 14**: PXRD pattern of a phase I form obtained by the evaporation *n*-butanol at -21 °C.
**Fig. 15**: PXRD pattern of a phase I form obtained by the slow crystallization of hot saturated P027 compound solution in methyl ethyl ketone.
**Fig. 16**: PXRD pattern of a phase I form obtained by crystallization through the addition of a P027 solution in methanol to an *n*-heptane solution.
**Fig. 17**: PXRD pattern of a phase I form obtained by crystallization through a liquid-liquid diffusion of a P027 solution in nitromethane and an isopropyl ether solution.
**Fig. 18**: PXRD pattern obtained after grinding a sample of P027 phase I form together with dichloromethane. The pattern is consistent with the standard phase I PXRD pattern demonstrating the phase stability.
**Fig. 19**: PXRD pattern of a sample of P027 phase I form after applying a pressure of 30 tons to the sample for 90 minutes. The pattern is consistent with the standard phase I PXRD pattern demonstrating the phase stability.

### DETAILED DESCRIPTION OF THE INVENTION

The inventors have found that the compound P027, which is the HCI salt of 4-[2-[[5-methyl-1-(2-naphthalenyl)-1 H-pyrazol-3-yl]oxy]ethyl]morpholine has advantages due to the fact that it is a crystalline solid, which simplifies isolation, purification and handling. In addition, the form I of this compound is stable and can be formulated and administered providing stable compositions and good pharmacological properties.

As used herein, the term "about" means a slight variation of the value specified, preferably within 10 percent of the value specified. Nevertheless, the term "about" can mean a higher tolerance of variation depending on for instance the experimental technique used. Said variations of a specified value are understood by the skilled person and are within the context of the present invention. Further, to provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

### A. Characterization of 4-[2-[[5-methyl-1-(2-naphthalenyl)-1 H-pyrazol-3-yl]oxy]ethyl]morpholine hydrochloride phase I crystalline form.

### 1) Equipment used

### a) Powder X-ray Diffraction analysis (PXRD)

Approximately 20 mg of the non manipulated samples were prepared in standard sample holders using two foils of polyacetate.

Powder diffraction patterns were acquired on a D8 Advance Series 2Theta/Theta powder diffraction system using Cu_{Kα}-radiation in transmission geometry. The system was equipped with a VÅNTEC-1 single photon counting PSD, a Germanium monochromator, a ninety positions auto changer sample stage, fixed divergence slits and radial soller. Programs used: Data collection with DIFFRAC plus XRD Commander V.2.5.1 and evaluation with EVA V.12.0.

### b) Proton Nuclear Magnetic Resonance (¹H NMR)

Proton nuclear magnetic resonance analyses were recorded in deuterated chloroform (CDCI₃) in a Bruker Avance 400 Ultrashield NMR spectrometer, equipped with a z-gradient 5 mm BBO (Broadband Observe) probe with ATM and an automatic BACS-120 autosampler. Spectra were acquired solving 2-10 mg of sample in 0.6 mL of deuterated solvent.

### c) Differential Scanning Calorimetry analysis (DSC)

Standard DSC analyses were recorded in a Mettler Toledo DSC822e. Samples of 1-2 mg were weighted into 40 µL aluminium crucibles with a pinhole lid, and were heated, under nitrogen (50 mL/min), from 30 to 300 °C at 10 °C/min. Data collection and evaluation was done with software STARe.

### d) Thermogravimetric analysis (TGA)

Thermogravimetric analyses were recorded in a Mettler Toledo SDTA851 e. Samples of 3-4 mg were weighted (using a microscale MX5, Mettler) into open 40 µL aluminium crucibles with a pinhole lid, and heated at 10 °C/min between 30 and 500 °C, under nitrogen (80 mL/min). Data collection and evaluation was done with software STARe.

### e) Fourier Transform Infrared analysis (FTIR)

The FTIR spectra were recorded using a Bruker Tensor 27, equipped with a MKII golden gate single reflection ATR system, a mid-infrared source as the excitation source and a DTGS detector. The spectra were acquired in 32 scans at a resolution of 4 cm⁻¹. No sample preparation was required to perform the analysis.

### f) Single Crystal X-Ray Diffraction analysis (SCXRD)

The measured crystals were selected using a Zeiss stereomicroscope using polarized light and prepared under inert conditions immersed in perfluoropolyether as protecting oil for manipulation. Crystal structure determination was carried out using a Bruker-Nonius diffractometer equipped with a APPEX 2 4K CCD area detector, a FR591 rotating anode with Mo_{Kα} radiation, Montel mirrors as monochromator and a Kryoflex low temperature device (T = 100 K). Fullsphere data collection omega and phi scans. Programs used: Data collection Apex2 V. 1.0-22 (Bruker-Nonius 2004), data reduction Saint + Version 6.22 (Bruker-Nonius 2001) and absorption correction SADABS V. 2.10 (2003). Crystal structure solution was achieved using direct methods as implemented in SHELXTL Version 6.10 (Sheldrick, Universtität Göttingen (Germany), 2000) and visualized using XP program. Missing atoms were subsequently located from difference Fourier synthesis and added to the atom list. Least-squares refinement on F₀² using all measured intensities was carried out using the program SHELXTL Version 6.10 (Sheldrick, Universtität Göttingen (Germany), 2000). All non hydrogen atoms were refined including anisotropic displacement parameters.

### 2) Results

The P027 phase I was characterized by powder X-ray diffraction (PXRD), proton nuclear magnetic resonance (¹H-NMR), differential scanning calorimetry (DSC), thermogravimetric analysis (TGA) and Fourier-transformed infrared spectroscopy. The present invention is directed in one aspect to the P027 phase I in itself, regardless of the technique used for its characterization. Therefore, the techniques and results provided herein are not intended to limit the present invention, but to serve as characterization of the same. The skilled person will be able, given the guidance and results described herein, to compare and characterize using the available techniques a crystalline phase form I of the compound 4-[2-[[5-methyl-1-(2-naphthalenyl)-1 H-pyrazol-3-yl]oxy]ethyl]morpholine hydrochloride (P027).

The P027 phase I form shows a PXRD pattern having characteristic peaks at a reflection angle [2θ] of about 5.9, 8.1, 11.3, 11.7, 14.2, 15.1, 15.8, 16.3, 16.8, 17.8, 18.1, 18.6, 19.8, 20.9, 21.9, 22.8, 23.0, 23.2, 23.6, 23.9, 24.3, 25.0, 25.1, 28.0, 28.3, 28.6, 29.0, 29.2, 30.7, and 30.9 with the 2θ values being obtained using copper radiation (C_{UKα1} 1.54060Å).

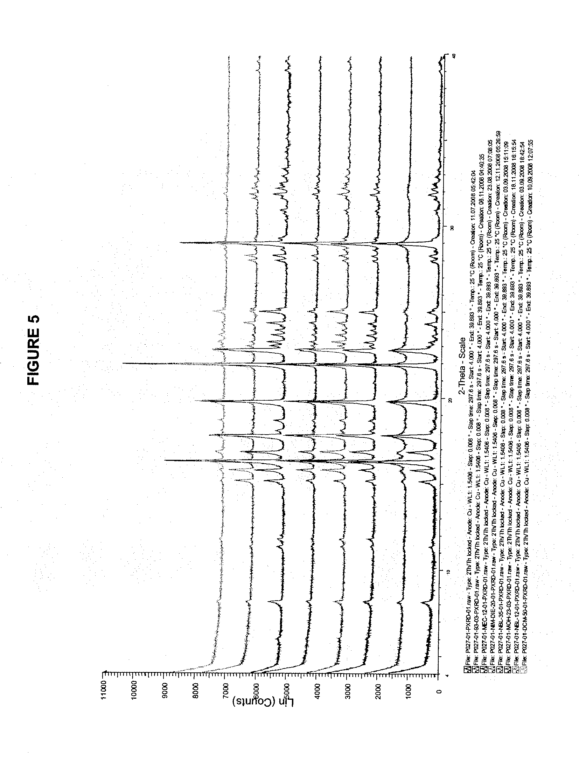

Differences in the PXRD patterns peak intensities could be observed depending of the crystallization procedure or crystallization solvent used (see Figure 5). Strong differences in the peak intensities could be due to preferred orientations, texture effects, of the crystals and are not indicative of the presence of different crystalline phases. Non ideal crystalline phases are defined by the peak positions and not by the peak intensities. Differences in the peak intensities could be due to different configurations of the measurement devices (transmission vs. reflection) or to texture effects related to the preferred orientations of the crystals.

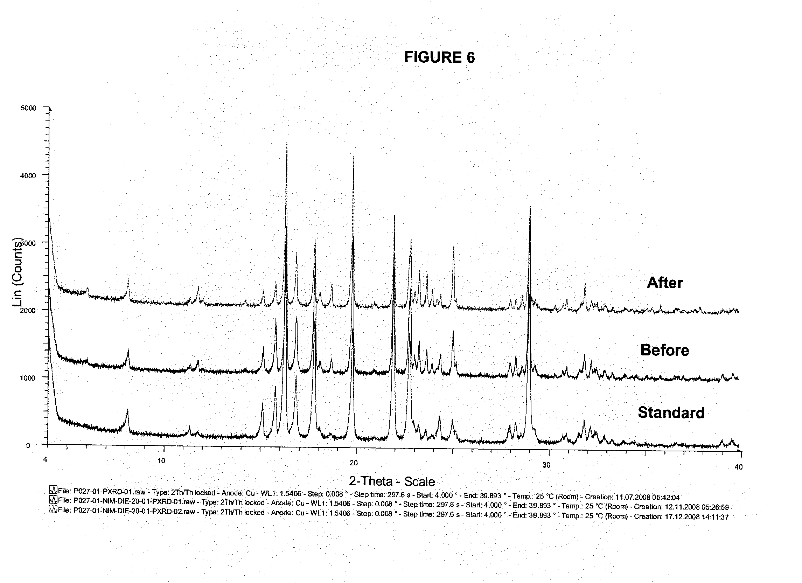

In order to verify if the differences in the peak intensities were due to texture effects, some selected samples were gently grinded in an agate mortar and measured. After homogenizing the samples, the texture effects became less pronounced or disappeared (see Figure 6).

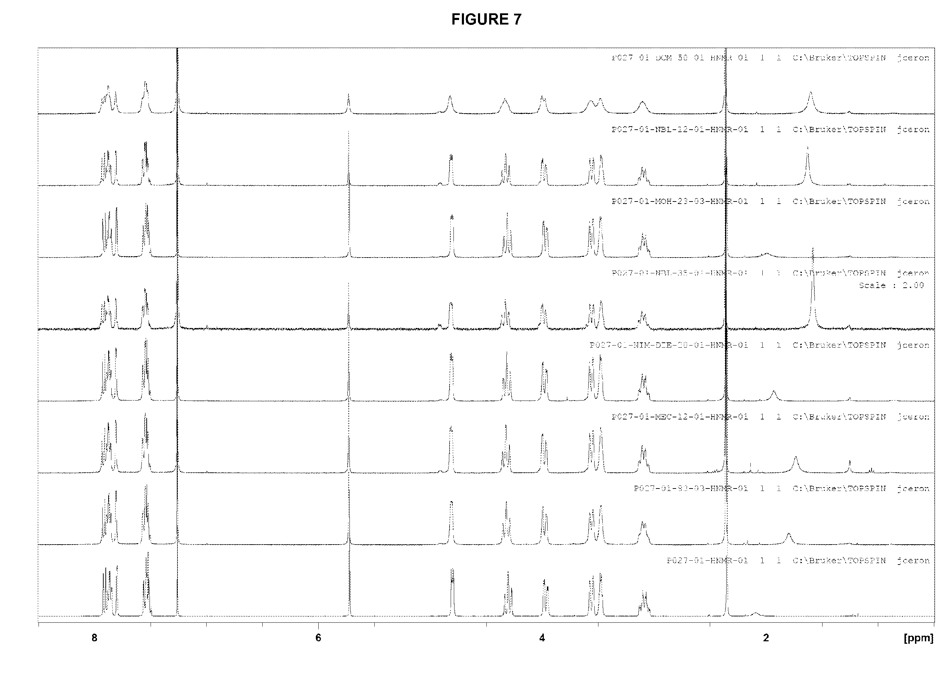

In addition, several samples of phase I were analyzed by ¹H NMR in order to check the stability of the salt. The chemical shifts and the integrations of the ¹H NMR signals were coincident for all samples and no signs of the lost of HCl or decomposition of the samples could be observed (see Figure 7).

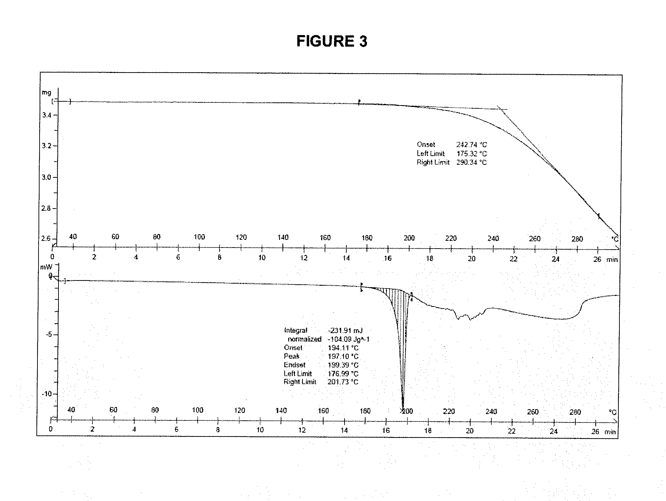

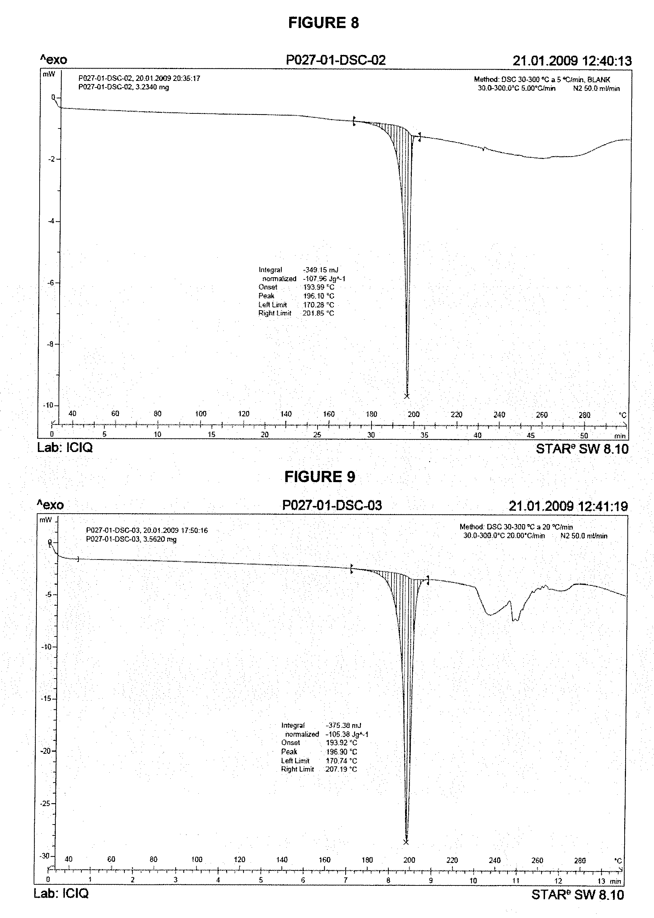

A DSC analysis of phase I samples was performed with a heating rate of 10 °C/min. The analysis presented a sharp endothermic peak, which does not recover the base line, with an onset at 194 °C and an enthalpy of 103 J/g corresponding to melting followed by decomposition of the product (see Figure 3). In additional DSC analyses of the same sample, performed with a heating rate of 5 °C/min and 20 °C/min, it was observed that the onset temperature of the endothermic peak does not vary with the heating rate (see Figures 8 and 9).

In a TGA of a phase I sample a weight loss, due to decomposition of the sample, was observed at temperatures higher than 195 °C (see Figure 3). No weight loss was observed at temperatures below 195 °C, indicating the absence of solvent. The onset temperature of weight loss in the TGA coincides with the melting temperature, confirming that the sample decomposes on melting.

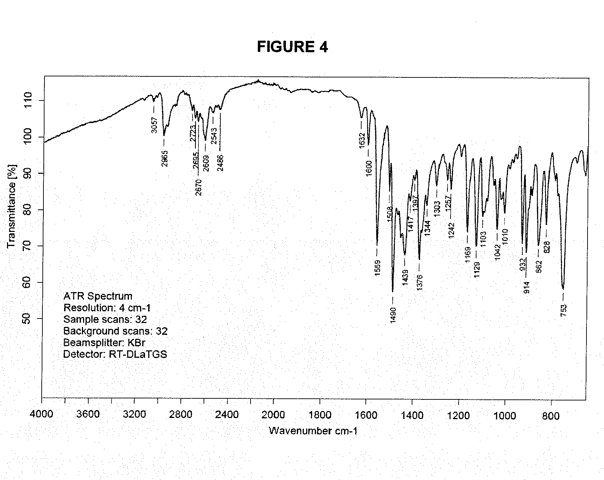

The FTIR spectrum of P027 phase I presented intense peaks at about 2965, 2609, 1632, 1600, 1559, 1508, 1490, 1439, 1376, 1301, 1257, 1242, 1169, 1129, 1103, 1042, 1010, 932, 914, 862, 828 and 753 cm⁻¹ (see Figure 4).

### B. Structure determination of 4-[2-[[5-methyl-1-(2-naphthalenyl)-1 H-pyrazol-3-yl]oxy]ethyl]morpholine hydrochloride phase I crystalline form by single crystal X-ray diffraction.

The identity and crystal structure of the P027 compound phase I was assayed by a single crystal X-ray structure determination. Suitable crystals were obtained by slow diffusion of *n*-heptane into a concentrated solution of the product in acetone. Since the selected crystals were mostly twinned, a small fragment of a plate (0.30 x 0.30 x 0.07 mm³) was separated with a micro scalpel and used for single crystal X-ray structure determination. Table 1 shows the measurement conditions utilized, cell constants and results obtained in a single crystal X-ray structure diffraction analysis. Table 2 depicts phase I selected bond distances and angles for an X-ray structure determination performed at 100 K.

**Table 1 Phase I single crystal X-ray structure diffraction analysis Measurement conditions, cell constants and results**

| **Parameter** | **Value** |
|---|---|
| Empirical formula | C20 H24 Cl1 N3 02 |
| Formula weight | 373.87 UMA |
| Temperature | 100(2) K |
| Wavelength | 0.71073 Å |
| Crystal system | Monoclinic |
| Space group | C2/c |
| Unit cell dimensions | a = 29.367(3) Å, α= 90° |
| | b = 11.6704(11) Å, β= 91.284(2)° |
| | c = 11.0437(10) Å, y = 90° |
| Volume | 3784.0(6) Å³ |
| z | 8 |
| Density (calculated) | 1.313 Mg/m³ |
| Absorption coefficient | 0.221 mm⁻¹ |
| F(000) | 1584 |
| Crystal size | 0.40 x 0.40 x 0.10 mm³ |
| Theta range for data collection | 2.64 to 38.06°. |
| Index ranges | -50<=h<=50,-20<=k<=18, -17<=l<=19 |
| Reflections collected | 42960 |
| Independent reflections | 10292 [R(int) = 0.0414] |
| Completeness to theta = 38.06° | 99.5 % |
| Absorption correction | SADABS (Bruker-Nonius) |
| Max. and min. transmission | 0.9782 and 0.9167 |
| Refinement method | Full-matrix least-squares on F² |
| Data / restraints / parameters | 10292 /0 /237 |
| Goodness-of-fit on F2 | 1.043 |
| Final R indices [l>2sigma(l)] | R1 = 0.0540, wR2 = 0.1409 |
| R indices (all data) | R1 = 0.0733, wR2 = 0.1586 |
| Largest diff. peak and hole | 2.152 and -1.013 e.Å⁻³ |

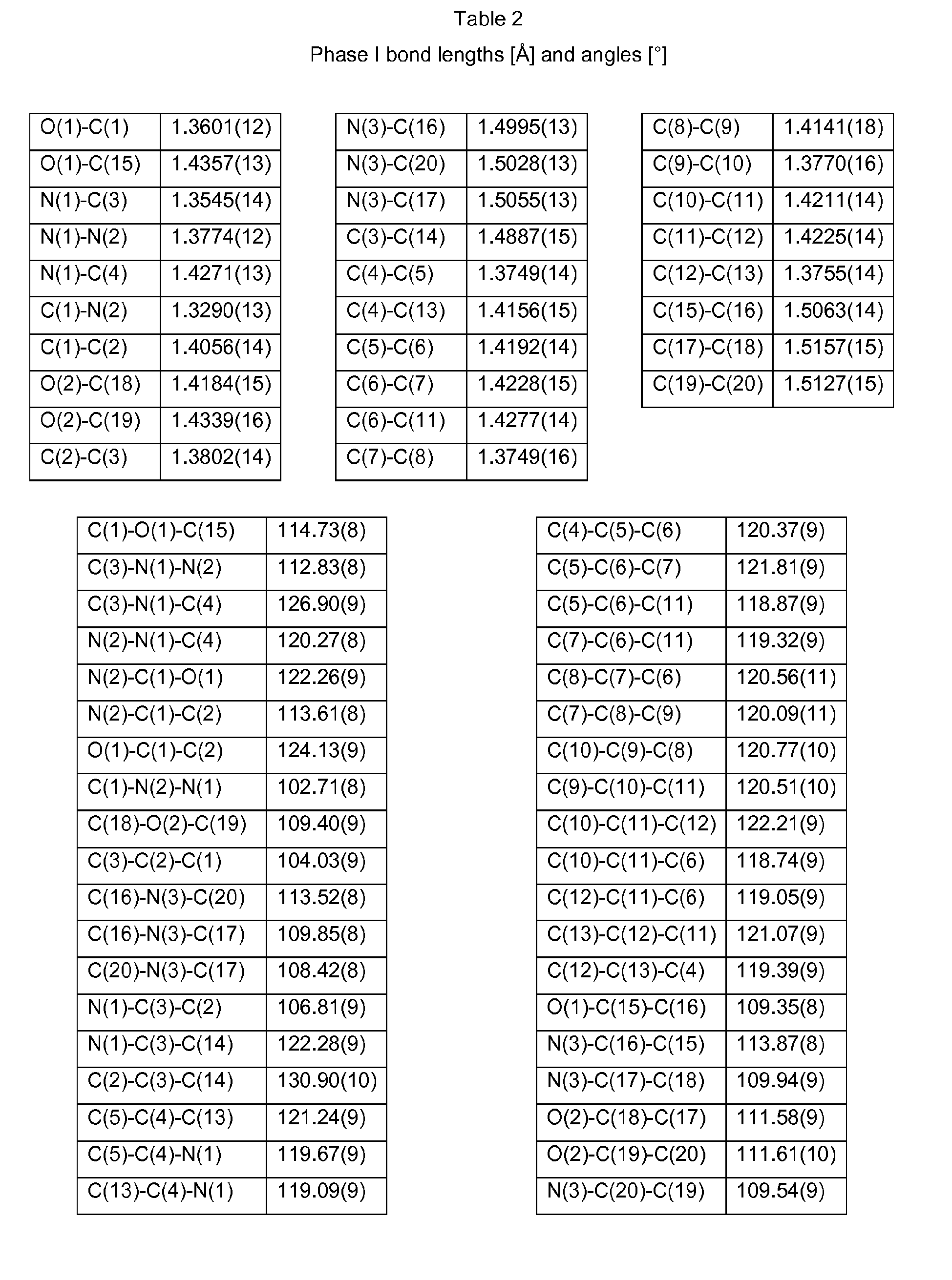

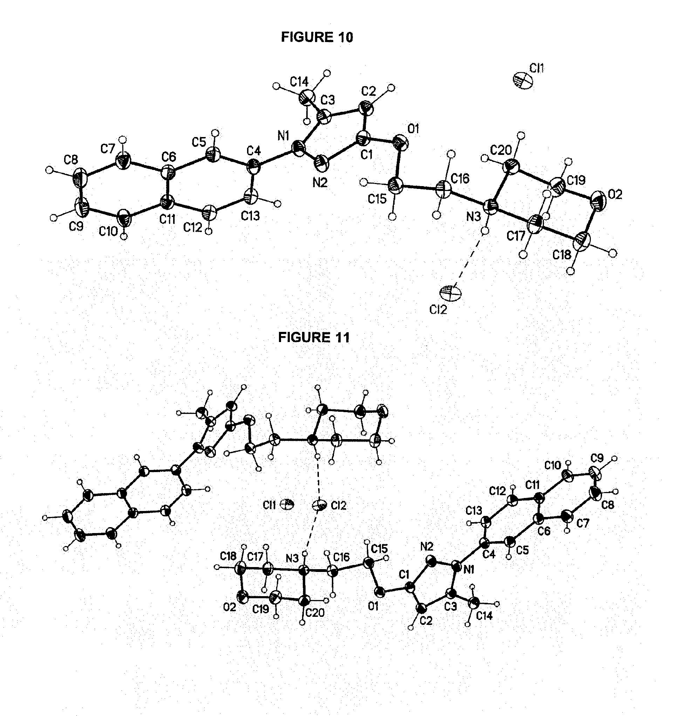

Phase I crystallizes in the centrosymmetric space C2/c with one cationic molecule and two half independent anionic chlorine atoms in the unit cell (see Figure 10). Each cationic molecule shares two chlorine anions with neighboring cationic molecules. One of the shared chlorine atoms is linked to the positively charged N-H-groups of two neighboring cationic molecules making two hydrogen bonds (Cl1 ... N3-distance: 3.13 A) [see Figures 10 and 11]. The second shared chlorine anion is located in the intermolecular space making only weak interactions to the surrounding molecules (shortest distance is C12...C17-distance: 3.56 Å).

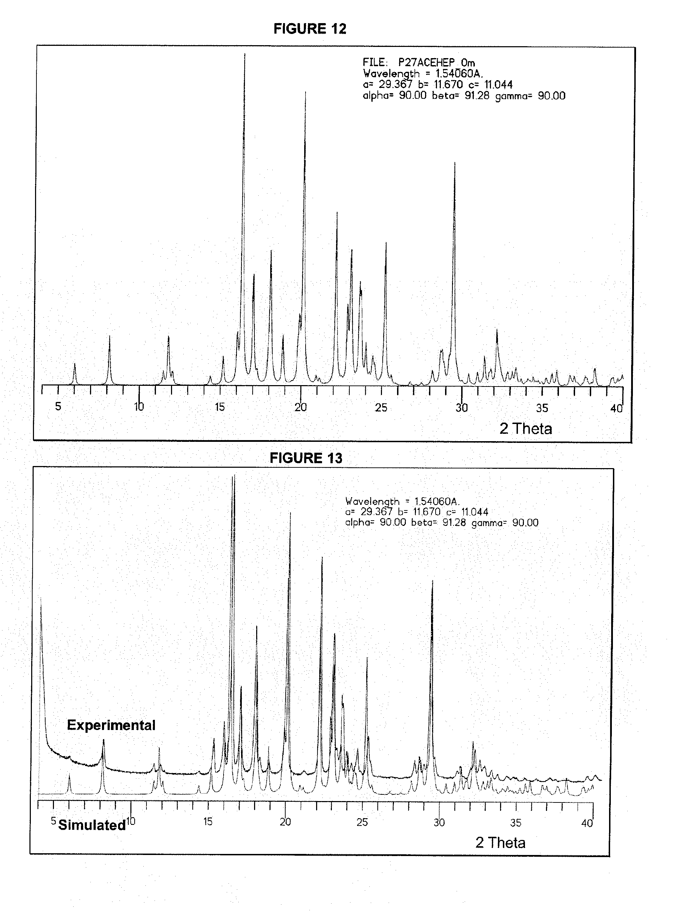

The powder diffraction pattern simulated from the single crystal data shows a good correspondence to the experimentally measured standard powder diffraction pattern of phase I. The overlay confirms the phase purity. Small variations in peak positions are due to the temperature difference at which the compared powder diffractograms were measured (simulated at -173 °C and experimentally measured at room temperature). Figures 12 and 13 show the phase I simulated powder diffraction pattern and its comparison to the experimentally measured pattern, respectively.

As used herein, "room temperature" or its abbreviation "rt" is taken to mean 20 to 25 °C.

### C. Preparation of 4-[2-[[5-methyl-1-(2-naphthalenyl)-1 H-pyrazol-3-yl]oxy]ethyl]morpholine hydrochloride phase I crystalline form

The preparation of P027 phase I form was attempted by utilizing solvents from the following list:

**Table 3 Solvents used in the crystallization of P027 phase I form**

| **Name** | **Code** | **Name** | **Code** |
|---|---|---|---|
| Dimethylsulfoxide | DMS | Diethoxymethane | DEM |
| N,N-Dimethylacetamide | DMA | 1,2-Dichloroethane | DCE |
| N,N-Dimethylformamide | DMF | Isopropanol | IPH |
| Xylene | XIL | Acetonitrile | ACN |
| Chlorobenzene | CLB | Cyclohexane | CHE |
| *n*-Butanol | NBL | Methyl ethyl ketone | MEC |
| Methyl isobutyl ketone | MIC | Butyl amine | BUA |
| Isobutyl acetate | AIB | Ethanol | EOH |
| Pyridine | PYR | Ethyl acetate | AET |
| Toluene | TOL | 1,1,1-Trichloroethane | TCE |
| 3-Pentanone | POA | n-Hexane | HEX |
| Propyl acetate | APR | Diisopropyl ether | DIE |
| Nitromethane | NIM | Tetrahydrofuran | THF |
| Dioxane | DIX | Methanol | MOH |
| Water | H2O | Chloroform | CLF |
| 2-Butanol | BUL | Methyl acetate | MAC |
| *n*-Heptane | HEP | Acetone | ACE |
| Dimethylcarbonate | CDM | Methyl *tert-*butyl ether | MTE |
| Triethylamine | TEA | Dimethoxymethane | DMM |
| Isopropyl acetate | AIP | Dichloromethane | DCM |

Initially, the solubility of the P027 compound in various Table 3 solvents was determined. The following values were obtained:

**Table 4 Solubility of P027 in different solvents at room temperature**

| **Solvent** | **mg/ml** | **Solvent** | **mg/ml** |
|---|---|---|---|
| Chloroform | > 50 | Dimethylcarbonate² | 1-2 |
| Dimethylsulfoxide | > 50 | Tetrahydrofuran | < 1.2 |
| Dimethylformamide | > 50 | Methyl acetate | < 1.2 |
| Dichloromethane | > 50 | Isobutyl acetate | < 1.2 |
| Methanol | > 50 | Propyl acetate | < 1.2 |
| Butyl amine | > 50 | Xylene | < 1.2 |
| Water | > 50 | Isopropyl acetate | < 1.2 |
| N,N-Dimethylacetamide | 25-50 | Toluene | < 1.2 |
| Nitromethane | 25-50 | Ethyl acetate | < 1.2 |
| Pyridine | 25-50 | 1,1,1-Trichloroethane | < 1.2 |
| Ethanol | 15-25 | Methyl isobutyl ketone | < 1.2 |
| 1,2-Dichloroethane | 15-25 | Methyl tert-butyl ether | < 1.2 |
| Acetonitrile¹ | 10-20 | Dimethoxymethane | < 1.2 |
| *n*-Butanol¹ | 5-10 | Cyclohexane | < 1.2 |
| Acetone | 4.0-5.0 | Chlorobenzene | < 1.2 |
| Isopropanol¹ | 4.0-5.0 | *n*-Heptane | < 1.2 |
| 2-Butanol¹ | 3-4 | *n*-Hexane | < 1.2 |
| Methyl ethyl ketone¹ | 2-4 | Diisopropyl ether | < 1.2 |
| 3-Pentanone² | 1-2 | Triethylamine | < 1.2 |
| Dioxane² | 1-2 | Diethoxymethane | < 1.2 |

| | | | |
|---|---|---|---|
| The solid was dissolved at 60 °C. The solution was left at room temperature and no solid was observed. ²The solid was dissolved at 80 °C. The solution was left at room temperature and no solid was observed. | | | |

The solvents where the P027 compound was insoluble were used as antisolvents (e.g. those solvents providing a solubility < 1.2 mg/mL). For example, *n*-Heptane (HEP), diisopropyl ether (DIE) and 2-methoxy-2-methylpropane (MMP) were used as antisolvents. The remaining agents were used as dissolving solvents in the different crystallization strategies assayed.

Several crystallization methodologies were employed using the solvents described in Table 3. The following procedures were performed to obtain the phase I form:
1) Solvent evaporation at two rates at room temperature
2) Solvent evaporation at different temperatures: -21, 4 and 60 °C
3) Crystallization from hot saturated solutions at two cooling rates
4) Crystallization by addition of an antisolvent
5) Crystallization by diffusion of an antisolvent
6) Crystallization from mixtures of water and solvents
7) Supensions

Therefore, any of such procedures can be regarded as a process to prepare the compound of the invention. Phase I was also tested under grinding and pressure conditions to evaluate its stability.

Having described the invention in general terms, it will be more easily understood by reference to the following examples which are presented as an illustration and are not intended to limit the present invention.

### EXAMPLES

### Example 1

### Initial synthesis and crystallization of the P027 compound

4-[2-[[5-methyl-1-(2-naphthalenyl)-1 H-pyrazol-3-yl]oxy]ethyl]morpholine hyd roch loride was obtained according to the following protocols:
1) 50.8 liters of a 6N hydrochloric acid/propan-2-ol solution was added to a solution of 4-[2-[[5-methyl-1-(2-naphthalenyl)-1 H-pyrazol-3-yl]oxy]ethyl]morpholine (85 kg) in ethanol (290 l) at T>35 °C. Then, 213 liters of 2-methoxy-2-methylpropane was added to the suspension. The mixture was cooled later at 0-5 °C. The resulting solid was isolated by centrifugation to yield 90 kg of the P027 compound.
2) 27 ml of a 6N hydrochloric acid/propan-2-ol solution was added to a solution of 4-[2-[[5-methyl-1-(2-naphthalenyl)-1 H-pyrazol-3-yl]oxy]ethyl]morpholine (44.5 g) in ethanol (120 mL) and 2-methoxy-2-methylpropane (112 mL) at T>35 °C. Next, the suspension was cooled at 0-5 °C. The resulting solid was isolated by filtration to yield 47 g of the P027 compound.

### Example 2

### Solvent evaporation at two rates at room temperature

Between 10 to 20 mg of the P027 compound were dissolved in the minimum amount of the relevant solvents at room temperature (rt), at 60 °C and 80 °C. The resulting solutions were left to evaporate rapidly in open vials or slowly in closed tubes pierced with a needle at room temperature (see Tables 5 and 6). Those solutions which were not completely evaporated after 3 months were allowed to evaporate at room temperature in open vials. The solid samples obtained were analyzed by PXRD. The samples showed a pattern consistent with the standard PXRD phase I pattern.

**Table 5 Fast solvent evaporation at room temperature**

| **Solvents** | **V (mL)** | **Dissolution temperature** |
|---|---|---|
| ACE | 2.5 | rt |
| ACN | 0.8 | 60 °C |
| BUL | 3.2 | 60 ºC |
| CDM | 8.0 | 80 ºC |
| CLF | 0.2 | rt |
| DCE | 0.6 | rt |
| DCM | 0.2 | rt |
| DIX | 7.0 | 80ºC |
| DMF | 0.2 | rt |
| EOH | 0.6 | rt |
| H2O | 0.2 | rt |
| IPH | 2.5 | 60 ºC |
| MEC | 3.5 | 60 ºC |
| MOH | 0.2 | rt |
| NBL | 1.4 | 60 ºC |
| NIM | 0.4 | rt |
| PYR | 0.4 | rt |

**Table 6 Slow solvent evaporation at room temperature**

| **Solvent** | **V (mL)** | **Dissolution temperature** |
|---|---|---|
| ACE | 2.5 | rt |
| ACN | 0.8 | 60 °C |
| CDM | 8.0 | 80 °C |
| CLF | 0.2 | rt |
| DCE | 0.6 | rt |
| DCM | 0.2 | rt |
| DMF | 0.2 | rt |
| EOH | 0.6 | rt |
| H2O | 0.2 | rt |
| IPH | 2.5 | 60 °C |
| MEC | 3.5 | 60 °C |
| NIM | 0.4 | rt |
| PYR | 0.4 | rt |

### Example 3

### Solvent evaporation at different temperatures

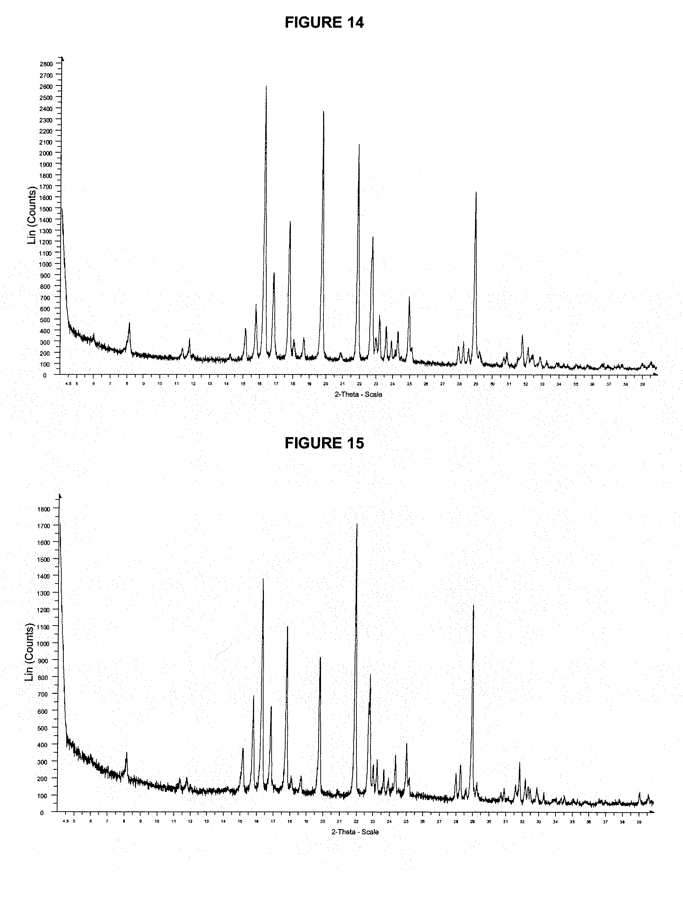

Between 10 to 20 mg of the P027 compound were dissolved in the minimum amount of the relevant solvents at room temperature (rt), at 60 °C or at 80 °C. The resulting solutions were left to evaporate, in open vials, at three different temperatures: 60 °C, 4 °C and -21 °C (see Tables 7, 8 and 9). Those solutions which were not completely evaporated after 3 months were allowed to evaporate at room temperature in open vials. The solid samples obtained were analyzed by PXRD. The samples showed a pattern consistent with the standard PXRD phase I pattern. Figure 14 illustrates the PXRD pattern of a phase I form obtained by the evaporation of a *n*-butanol solution at - 21 °C according to the present protocol.

**Table 7 Solvent evaporation at 60 °C**

| **Solvent** | **V (mL)** | **Dissolution temperature** |
|---|---|---|
| ACE | 2.5 | rt |
| ACN | 0.8 | 60 °C |
| BUL | 3.2 | 60 °C |
| CDM | 8.0 | 80 °C |
| CLF | 0.2 | rt |
| DCE | 0.6 | rt |
| DCM | 0.2 | rt |
| DIX | 7.0 | 80 °C |
| DMA | 0.4 | rt |
| DMF | 0.2 | rt |
| DMS | 0.2 | rt |
| EOH | 0.6 | rt |
| H2O | 0.2 | rt |
| IPH | 2.5 | 60 °C |
| MEC | 3.5 | 60 °C |
| MOH | 0.2 | rt |
| NBL | 1.4 | 60 °C |
| NIM | 0.4 | rt |
| POA | 6.0 | 80 °C |
| PYR | 0.4 | rt |

**Table 8 Solvent evaporation at 4°C**

| **Solvent** | **V(mL)** | **Dissolution temperature** |
|---|---|---|
| ACE | 2.5 | rt |
| ACN | 0.8 | 60 °C |
| CLF | 0.2 | rt |
| DCE | 0.6 | rt |
| DCM | 0.2 | rt |
| DIX¹ | 7.0 | 80 °C |
| DMF¹ | 0.2 | rt |
| EOH | 0.6 | rt |
| IPH | 2.5 | 60 °C |
| MEC | 3.5 | 60 °C |
| MOH | 0.2 | rt |
| NBL¹ | 1.4 | 60 °C |
| NIM | 0.4 | rt |
| POA¹ | 6.0 | 80 °C |
| PYR | 0.4 | rt |

| | | |
|---|---|---|
| ¹The solution was left to evaporate in an open vial at room temperature. | | |

**Table 9 Solvent evaporation at -21 °C**

| **Solvent** | **V(mL)** | **Dissolution temperature** |
|---|---|---|
| ACE | 2.5 | rt |
| ACN | 0.8 | 60 °C |
| BUL | 3.2 | 60 °C |
| CLF | 0.2 | rt |
| DCE | 0.6 | rt |
| DCM | 0.2 | rt |
| DMF | 0.2 | rt |
| EOH¹ | 0.6 | rt |
| IPH | 2.5 | 60 °C |
| MEC¹ | 3.5 | 60 °C |
| MOH | 0.2 | rt |
| NBL | 1.4 | 60 °C |
| NIM | 0.4 | rt |
| PYR¹ | 0.4 | rt |

| | | |
|---|---|---|
| ¹The solution was left to evaporate in an open vial at room temperature. | | |

### Example 4

### Crystallization from hot saturated solutions

Between 20 to 30 mg of the P027 compound were dissolved in the minimum amount of the relevant solvents at high temperature to obtain saturated solutions. The solutions were then cooled by two different methods:
1) Slow cool down at room temperature (slow crystallization) [see Table 10].
2) Rapid cool down by ice bath immersion (fast crystallization) [see Table 11].

After cooling at room temperature the solids obtained were separated by filtration or centrifugation. If no solids were formed, the solution was kept at 4 °C for a few days in first step. Any solids formed during this step were separated from the solution. If no solids were formed during the first step, the solution was kept at -21 °C for a few additional days. Any solids formed during this second step were separated from the solution. The solutions that did not crystallize during the second step were left to evaporate to dryness at room temperature. The solid was filtered off in some experiments when crystallization occurred before complete evaporation.

The solid samples obtained were analyzed by PXRD. The samples showed a pattern consistent with the standard PXRD phase I pattern. Figure 15 illustrates the PXRD pattern of a phase I form obtained by the slow crystallization of hot saturated P027 compound solution in methyl ethyl ketone.

**Table 10 Slow crystallization from hot saturated solutions**

| **Solvent** | **V(mL)** | **Dissolution temperature** | **Crystalliztion conditions** |
|---|---|---|---|
| ACE | 3.7 | 56 °C | at -21 °C |
| ACN | 0.6 | 80 °C | at rt |
| BUL | 0.4 | 98 °C | at rt |
| CLF | 0.2 | rt | Evaporation |
| DCE | 0.4 | 80 °C | at rt |
| DCM | 0.4 | 40 °C | Evaporation |
| DIX | 1.0 | 101 °C | at rt |
| DMA | 0.2 | 100 °C | at -21 °C |
| DMF | 0.2 | 100 °C | Evaporation |
| EOH | 0.4 | 78 °C | at rt |
| H2O | 0.2 | 100 °C | Evaporation |
| IPH | 0.6 | 80 °C | at rt |
| MEC | 2.7 | 80 °C | at 4 °C |
| MOH | 0.2 | 56 °C | at -21 °C |
| NBL | 0.4 | 118 °C | at rt |
| NIM | 0.4 | 101 °C | at -21 °C |
| PYR | 0.2 | rt | Evaporation |

**Table 11 Fast crystallization from hot saturated solutions**

| **Solvent** | **V(mL)** | **Dissolution temperature** | **Crystalliztion conditions** |
|---|---|---|---|
| ACE | 4.0 | 56 °C | Immediately |
| ACN | 0.6 | 80 °C | at -21 °C |
| BUL | 0.4 | 98 °C | Immediately |
| CLF | 0.2 | rt | Evaporation |
| DCE | 0.4 | 80 °C | at -21 °C |
| DCM | 0.4 | 40 °C | at -21 °C |
| DIX | 1.0 | 101 °C | Immediately |
| DMA | 0.2 | 100 °C | Evaporation |
| DMF | 0.2 | 100 °C | Evaporation |
| EOH | 0.4 | 78 °C | at -21 °C |
| H2O | 0.2 | rt | Evaporation |
| IPH | 0.6 | 80 °C | at -21 °C |
| MEC | 2.7 | 80 °C | at -21 °C |
| MOH | 0.2 | 56 °C | at -21 °C |
| NBL | 0.4 | 118 °C | Immediately |
| NIM | 0.4 | 101 °C | Immediately |
| PYR | 0.2 | rt | Immediately |

### Example 5

### Small scale crystallization by addition of an antisolvent

Between 10 to 20 mg of the P027 compound were dissolved in the minimum amount of the relevant dissolving agent at high temperature or at room temperature. Diisopropyl ether (DIE) and *n*-heptane (HEP) were used as antisolvents. The following protocols were performed:
1) The antisolvent was added drop wise to a P027 solution under vigorous stirring at room temperature or at high temperature (see Tables 12 and 13).
2) A P027 solution was added drop wise to 4 mL of the antisolvent under vigorous stirring at room temperature or at high temperature (see Tables 14 and 15).

The solids obtained after mixing the dissolving agent and antisolvent were separated from the solution by filtration or centrifugation. If no solids were formed, the solution was kept at 4 °C for a few days in first step. Any solids formed during this step were separated from the solution. If no solids were formed during the first step, the solution was kept at -21 °C for a few additional days. Any solids formed during this second step were separated from the solution. The solutions that did not crystallize during the second step were left to evaporate to dryness at room temperature. The solid was filtered off in some experiments when crystallization occurred before complete evaporation.

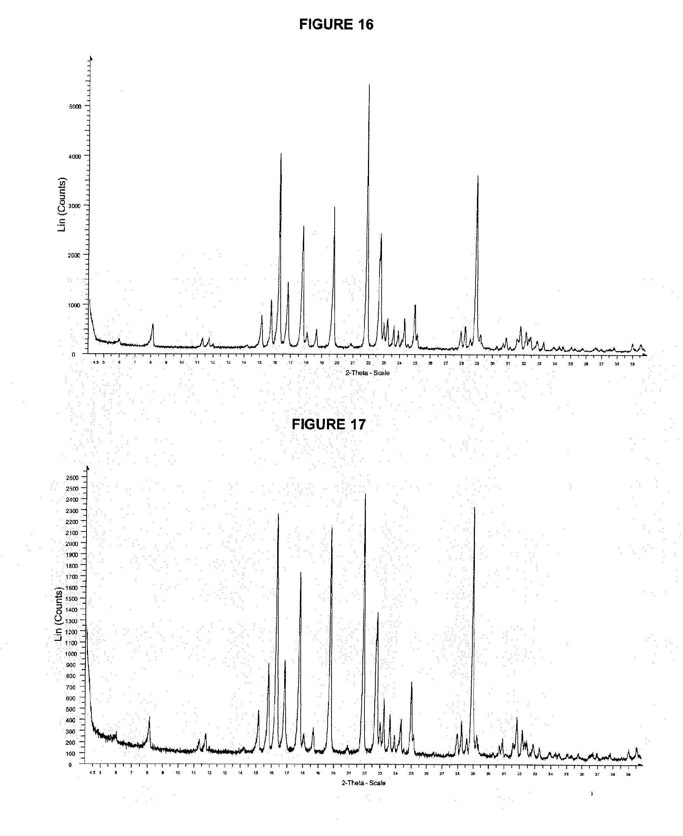

The solid samples obtained were analyzed by PXRD. The samples showed a pattern consistent with the standard PXRD phase I pattern. Figure 16 illustrates the PXRD pattern of a phase I form obtained by crystallization through the addition of a P027 solution in methanol to an *n*-heptane solution.

**Table 12 Crystallization by the addition of an antisolvent to a P027 solution at room temperature**

| **Dissolving Solvent** | **Anti solvents** | **Crystallization conditions** |
|---|---|---|
| ACE | DIE | at -21°C |
| | HEP | Evaporation |
| ACN | DIE | Immediately |
| | HEP¹ | at 4°C |
| BUL | DIE¹ | Immediately |
| | HEP¹ | Immediately |
| CLF | DIE | Immediately |
| | HEP | Immediately |
| DCE | DIE | Immediately |
| | HEP | Immediately |
| DCM | DIE | Immediately |
| | HEP | Immediately |
| DIX | DIE¹ | Immediately |
| | HEP | Immediately |
| DMA | DIE | Immediately |
| | HEP¹ | Evaporation |
| EOH | DIE | Immediately |
| | HEP | Immediately |
| IPH | DIE | at 4°C |
| | HEP | at 4°C |
| MEC | DIE | Immediately |
| | HEP | Immediately |
| MOH | DIE | Immediately |
| | HEP¹ | Immediately |
| NBL | DIE | Immediately |
| | HEP¹ | Immediately |
| NIM | DIE | Immediately |
| | HEP | Evaporation |
| PYR | DIE | Immediately |
| | HEP | Immediately |

| | | |
|---|---|---|
| ¹Solvent and antisolvent were immiscible. | | |

**Table 13 Crystallization by the addition of an antisolvent to a P027 solution at high temperature**

| **Dissolving solvent** | **T (°C)** | **Antisolvent** | **Crystallization conditions** |
|---|---|---|---|
| ACN | 65 | DIE | Immediately |
| | 80 | HEP | at -21°C |
| BUL | 65 | DIE | Immediately |
| | 90 | HEP | Immediately |
| CLF | 60 | DIE | Immediately |
| | 60 | HEP | Immediately |
| DCE | 65 | DIE | Immediately |
| | 80 | HEP | Immediately |
| DCM | 40 | DIE | Immediately |
| | 40 | HEP | Immediately |
| DIX | 65 | DIE | Immediately |
| | 90 | HEP | at -21°C |
| DMA | 65 | DIE | Immediately |
| | 90 | HEP | Evaporation |
| EOH | 65 | DIE | Immediately |
| | 75 | HEP | Immediately |
| IPH | 65 | DIE | at -21 °C |
| | 80 | HEP | at -21 °C |
| MEC | 65 | DIE | Immediately |
| | 80 | HEP | Immediately |
| MOH | 65 | DIE | Immediately |
| | 65 | HEP | Immediately |
| NBL | 65 | DIE | Immediately |
| | 90 | HEP | Immediately |
| NIM | 65 | DIE | Immediately |
| | 90 | HEP | Evaporation |

**Table 14 Crystallization by the addition of a P027 solution to an antisolvent at room temperature**

| **Dissolving** | **Antisolvent** | **Crystallization conditions** |
|---|---|---|
| ACE | DIE | Immediately |
| | HEP | Immediately |
| ACN | DIE | Immediately |
| | HEP¹ | Immediately |
| BUL | DIE | Immediately |
| | HEP | Immediately |
| CLF | DIE | Immediately |
| | HEP | Immediately |
| DCE | DIE | Immediately |
| | HEP | Immediately |
| DCM | DIE | Immediately |
| | HEP | Immediately |
| DIX | DIE | Immediately |
| | HEP | Immediately |
| DMA | DIE | Immediately |
| | HEP¹ | Immediately |
| EOH | DIE | Immediately |
| | HEP | Immediately |
| IPH | DIE | Immediately |
| | HEP | Immediately |
| MEC | DIE | Immediately |
| | HEP | Immediately |
| MOH | DIE | Immediately |
| | HEP¹ | Evaporation |
| NBL | DIE | Immediately |
| | HEP¹ | Immediately |
| NIM | DIE | Immediately |
| | HEP¹ | Evaporation |

| | | |
|---|---|---|
| ¹Solvent and antisolvent were immiscible. | | |

**Table 15 Crystallization by the addition of a P027 solution to an antisolvent at high temperature**

| **Dissolving solvent** | **T(ºC)** | **Antisolvent** | **Crystallization conditions** |
|---|---|---|---|
| ACE | 55 | DIE | Immediately |
| | 55 | HEP | Immediately |
| ACN | 65 | DIE | Immediately |
| | 80 | HEP¹ | at 4°C |
| BUL | 65 | DIE | at 4°C |
| | 90 | HEP | Immediately |
| CLF | 60 | DIE | Immediately |
| | 60 | HEP | Immediately |
| DCE | 65 | DIE | Immediately |
| | 80 | HEP | Immediately |
| DCM | 40 | DIE | Immediately |
| | 40 | HEP | Immediately |
| DMA | 65 | DIE | Immediately |
| | 90 | HEP | Immediately |
| EOH | 65 | DIE | Immediately |
| | 75 | HEP | Immediately |
| IPH | 65 | DIE | at 4°C |
| | 80 | HEP | Immediately |
| MEC | 65 | DIE | Immediately |
| | 80 | HEP | Immediately |
| MOH | 65 | DIE | Immediately |
| | 65 | HEP¹ | Evaporation |
| NBL | 65 | DIE | at 4°C |
| | 90 | HEP | Immediately |
| NIM | 65 | DIE | Immediately |
| | 90 | HEP¹ | Evaporation |

| | | | |
|---|---|---|---|
| ¹Solvent and antisolvent were immiscible. | | | |

### Example 6

### Large scale crystallization by addition of an antisolvent

133 liters of 2-methoxy-2-methylpropane was added to a P027 compound (45 kg) in ethanol (265 l) solution of at T>35 °C. Next, the suspension was cooled at 0-5 °C. The resulting solid was isolated by centrifugation to yield 40.2 kg of 4-[2-[[5-methyl-1-(2-naphthalenyl)-1 H-pyrazol-3-yl]oxy]ethyl]morpholine hydrochloride.

### Example 7

### Crystallization by diffusion of an antisolvent

Between 10 to 50 mg of the P027 compound were dissolved in the minimum amount of the relevant solvents at high temperature or at room temperature. Various dissolving agents were utilized. The following protocols were performed:
1) Liquid-liquid diffusion. The antisolvent was added carefully over a P027 solution forming two separated phases. The solid crystallized due to the diffusion of the phases (see Table 16).
2) Gas-liquid diffusion. A first container with a P027 solution was inserted into a second larger recipient containing the antisolvent. The gas diffusion of the antisolvent over the P027 solution induced the crystallization of phase I (see Table 17).

The solid samples obtained were analyzed by PXRD. The samples showed a pattern consistent with the standard PXRD phase I pattern. Figure 17 illustrates the PXRD pattern of a phase I form obtained by crystallization through a liquid-liquid diffusion of isopropyl ether into a P027 solution in nitromethane.

**Table 16 Crystallization by liquid-liquid diffusion**

| **Dissolving Solvent** | **V solvent (mL)¹** | **Antisolvent** | **Crystallization conditions** |
|---|---|---|---|
| ACN | 0.8 | DIE | Crystallization |
| | | HEP | Crystallization |
| CLF | 1 | DIE | Crystallization |
| | | HEP | Crystallization |
| DCE | | DIE | Crystallization |
| | 1.2 | HEP | Crystallization |
| DCM | 1 | DIE | Crystallization |
| | | HEP | Crystallization |
| EOH | 1.2 | DIE | Crystallization |
| | | HEP | Crystallization |
| IPH | 2.5 | DIE | Evaporation |
| | | HEP | Crystallization |
| MOH | 1 | DIE | Evaporation |
| | | HEP | Evaporation |
| NIM | 1 | DIE | Evaporation |
| | | HEP | Evaporation |
| PYR | 1 | DIE | Evaporation |
| | | HEP | Crystallization |

| | | | |
|---|---|---|---|
| ¹Equal quantities of dissolving solvent and antisolvent were added. | | | |

**Table 17 Crystallization by gas-liquid diffusion**

| **Dissolving solvent** | **Antisolvent** | **Crystallization conditions** |
|---|---|---|
| ACN | HEP | Evaporation |
| | DIE | Crystallization |
| CLF | HEP | Crystallization |
| | DIE | Crystallization |
| DCE | HEP | Crystallization |
| | DIE | Crystallization |
| DCM | HEP | Crystallization |
| | DIE | Crystallization |
| DMF | HEP | Crystallization |
| | DIE | Crystallization |
| EOH | HEP | Crystallization |
| | DIE | Crystallization |
| MOH | HEP | Crystallization |
| | DIE | Crystallization |
| NIM | HEP | Evaporation |
| | DIE | Evaporation |
| PYR | HEP | Crystallization |
| | DIE | Crystallization |

### Example 8

### Crystallization from mixtures of water and solvent

Between 10 to 20 mg of the P027 compound were dissolved in the minimum amount of the relevant solvent saturated with water. The solvents were mixed at various ratios with water according to their miscibility (see Table 18).

The solutions were allowed to crystallize at room temperature in a closed tube for two weeks. If no solids were formed, the solution was kept at 4 °C for a few days. Any solids formed during this step were separated from the solution. If no solids were formed during the first step, the solution was left to evaporate to dryness at room temperature.

The solid samples obtained were analyzed by PXRD. The samples showed a pattern consistent with the standard PXRD phase I pattern.

**Table 18 Crystallization from mixtures of water and solvent**

| **solvent** | **V(ml)** | **Dissolution Temperature (C°)** | **Water saturation** | **Observations** |
|---|---|---|---|---|
| ACN | 0.2 | 60 | 0 % | Evaporation |
| | 0.2 | 60 | 75 % | Evaporation |
| CLF | 0.9 | 60 | 0.2 % | Evaporation |
| DCE | 0.4 | 70 | 0.15 % | Evaporation |
| DCM | 0.4 | 40 | 0.15 % | Evaporation |
| DIX | 0.2 | 60 | 50 % | Evaporation |
| | 0.2 | 60 | 75 % | Evaporation |
| IPH | 0.2 | 60 | 25 % | Evaporation |
| | 0.2 | 60 | 50 % | Evaporation |
| MEC | 0.2 | 60 | 10 % | Evaporation |
| MOH | 0.2 | 60 | 25 % | Evaporation |
| NBL | 0.2 | 60 | 15 % | Crystallization |

### Example 9

### Grinding

Approximately 40 mg of P027 phase I were transferred to a ball mill container together with catalytic quantities of the relevant solvent (three drops). The P027 phase I and the solvent were grinded at a maximum frequency of 30 s⁻¹ for 30 minutes (see Table 19).

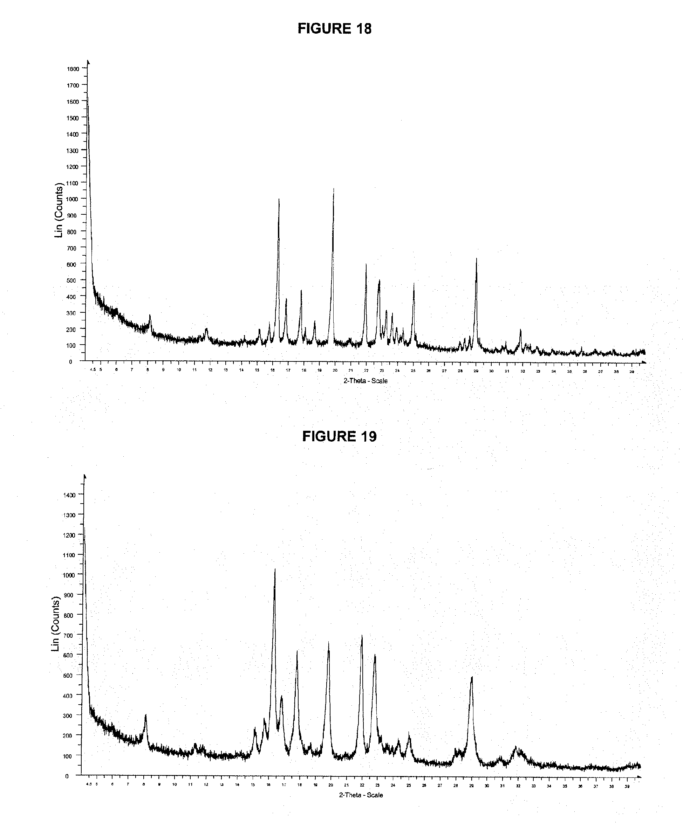

The solid samples obtained were analyzed by PXRD. The samples showed a pattern consistent with the standard PXRD phase I pattern, thus demonstrating that P027 phase l is stable after grinding. Figure 18 illustrates the PXRD pattern of a phase I form obtained from grinding P027 together with dichloromethane.

**Table 19 Solvents utilized in grinding assays**

| | | | |
|---|---|---|---|
| ACE | DCE | EOH | NIM |
| ACN | DCM | IPH | POA |
| BUL | DMA | MEC | PYR |
| CDM | DMF | MOH | THF |
| CLF | DMS | NBL | |

### Example 10

### Pressure

Tablets of P027 phase I were prepared in a hydraulic press at three different pressures (5, 7.5 and 10 tons) for three different times (5, 30 and 90 minutes) [see Table 20].

The solid samples obtained were analyzed by PXRD. The samples showed a pattern consistent with the standard PXRD phase I pattern, thus demonstrating that P027 phase I is stable under pressure. Figure 19 illustrates the PXRD pattern of a phase I form obtained by applying a pressure of 30 tons to P027 for 90 minutes.

**Table 20 Pressure parameters**

| **Pressure (Tons)** | **Time (min)** |
|---|---|
| 5 | 5 |
| 5 | 30 |
| 5 | 90 |
| 7.5 | 5 |
| 7.5 | 30 |
| 7.5 | 90 |
| 10 | 5 |
| 10 | 30 |
| 10 | 90 |

### Example 11

### Preparation of suspensions

Between 30 to 400 mg of the P027 compound were stirred in 4 mL of the relevant solvent for: i) 48 hours at room temperature or ii) 24 hours at 80 °C (see Table 21).

All suspensions were filtered. The solid samples obtained were analyzed by PXRD. The samples showed a pattern consistent with the standard PXRD phase I pattern.

**Table 21 Slurry suspensions**

| **Solvent** | **T (°C)** |
|---|---|
| DIE | rt |
| | 65 |
| HEP | rt |
| | 80 |
| TCE | rt |
| | 70 |

## Claims

1. A hydrochloride salt of 4-[2-[[5-methyl-1-(2-naphthalenyl)-1 H-pyrazol-3-yl]oxy]ethyl]morpholine.

2. A crystalline form of the hydrochloride salt according to claim 1.

3. The crystalline form according to claim 2, having a X-ray powder diffraction pattern showing characteristic peaks at a reflection angle [2θ] of about 5.9, 8.1, 11.3, 11.7, 14.2, 15.1, 15.8, 16.3, 16.8, 17.8, 18.1, 18.6, 19.8, 20.9, 21.9, 22.8, 23.0, 23.2, 23.6, 23.9, 24.3, 25.0, 25.1, 28.0, 28.3, 28.6, 29.0, 29.2, 30.7, and 30.9 with the 2θ values being obtained using copper radiation (CU_{Kα1} 1.54060Å).

4. A process for the preparation of the hydrochloride salt of claim 1, comprising:
a) mixing 4-[2-[[5-methyl-1-(2-naphthalenyl)-1 H-pyrazol-3-yl]oxy]ethyl]morpholine and a solution containing hydrochloric acid, and
b) isolating the resulting hydrochloride salt.

5. A process for the preparation of the crystalline form of any of claims 2 or 3, comprising:
a) dissolving 4-[2-[[5-methyl-1-(2-naphthalenyl)-1 H-pyrazol-3-yl]oxy]ethyl]morpholine hydrochloride in a suitable solvent, and
b) evaporating the solvent.

6. The process according to claim 5, wherein 4-[2-[[5-methyl-1-(2-naphthalenyl)-1H-pyrazol-3-yl]oxy]ethyl]morpholine hydrochloride is dissolved at a temperature ranging from room temperature to 120 °C.

7. The process according to claim 5, wherein the solvent is evaporated at a temperature ranging from -21 °C to 60 °C.

8. A process for the preparation of the crystalline according to any of claims 2 or 3, wherein a solution comprising 4-[2-[[5-methyl-1-(2-naphthalenyl)-1H-pyrazol-3-yl]oxy]ethyl]morpholine hydrochloride and a suitable antisolvent are mixed.

9. The process according to claim 8, wherein the mixture is performed at a temperature ranging from room temperature to 90 °C.

10. The process according to claim 8, wherein the mixture is performed by a liquid-liquid diffusion.

11. The process according to claim 8, wherein the mixture is performed by a gas-liquid diffusion.

12. The process according to claim 5, wherein water is added to the solution.

13. A process for the preparation of the crystalline form according to any of claims 2 or 3, wherein a suspension comprising 4-[2-[[5-methyl-1-(2-naphthalenyl)-1 H-pyrazol-3-yl]oxy]ethyl]morpholine hydrochloride is prepared.

14. The process according to claim 13, wherein the suspension is maintained at a temperature ranging from room temperature to 80 °C.

15. A pharmaceutical composition comprising the hydrochloride salt of claim 1.

16. A pharmaceutical composition comprising the crystalline form of any of claims 2 or 3.
